# EUROPEAN PATENT APPLICATION

(11) **EP 3 599 771 A2**
(43) Date of publication of application: **29.01.2020**
(21) Application number: 19184882.9
(22) Date of filing: 08.07.2019
(51) Int. Cl.: H04R 1/10, A61F 11/08

(54) **HEARING PROTECTION DEVICE AND METHOD**

(30) Priority: 24.07.2018 HK 18109577
(71) Applicant: HearSafe Limited, Kowloon, Hong Kong (HK)
(72) Inventor: CHEUNG, Yat Yiu, Hong Kong (HK)
(74) Representative: Marks & Clerk (Luxembourg) LLP

(57) **Abstract**

A hearing protection device (100) is disclosed as including at least a first audio signal processor (P₁) with a first audio signal frequency filter (F₁) and a first audio signal compressor (C₁) and a second audio signal processor (Pₙ) with a second audio signal frequency filter (Fₙ) and a second audio signal compressor (Cₙ), the first audio signal processor and the second audio signal processor being arranged in parallel with one another, the first audio signal processor being adapted to pass audio signals within a first frequency range and to compress the audio signals within the first frequency range when the volume of the audio signals within the first frequency range is above a first threshold volume, and the second audio signal processor being adapted to pass audio signals within a second frequency range and to compress the audio signals within the second frequency range when the volume of the audio signals within the second frequency range is above a second threshold volume.

## Description

This invention relates to a hearing protection device and a hearing protection method, in particular such a device and method suitable for reducing the level of noise heard by a user.

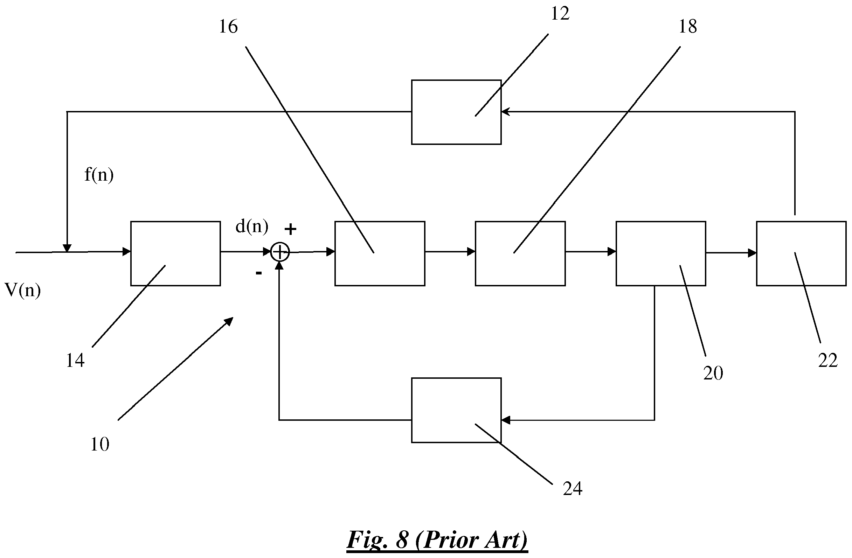

Fig. 8 shows a schematic diagram of a circuit of a prior art hearing protection device, generally designated as 10. A microphone 14 of the hearing protection device 10 picks up outside sound and feeds the sound, *via* a delay module 16, to a filter 18 for filtering out unwanted noise. The thus filtered sound is then fed to an output compression limit unit 20 for compression purposes. The compressed sound signals are then fed to a speaker 22 for output. A part of the compressed sound is fed back to the circuit of the hearing protection device 10 *via* an adapter filer 24. Apart of the sound outputted by the speaker 22 is also fed back to the microphone 14 *via* an acoustic feedback unit 12.

A disadvantage associated with this prior art hearing protection device 10 is that all frequencies of the sound received by the hearing protection device 10 undergo the same process. However, it is well known that different people have different noise reduction requirements, depending on the noise level of their usual living environment, working environment, or entertainment environment. In addition, even for a certain individual, the noise reduction requirements may differ according to the specific environment which he/she is in. Furthermore, a healthy individual may have different noise reduction preference/requirement across different frequency ranges (which may also be called "bands") of the humanly audible range.

It is thus an object of the present invention to provide a protection hearing device and a hearing protection method in which the aforesaid shortcomings are mitigated or at least to provide a useful alternative to the trade and public.

According to a first aspect of the present invention, there is provided a hearing protection device including at least a first audio signal processor and a second audio signal processor arranged in parallel with one another, wherein said first audio signal processor is adapted to pass audio signals within a first frequency range and to compress said audio signals within said first frequency range when the volume of said audio signals within said first frequency range is above a first threshold volume, and wherein said second audio signal processor is adapted to pass audio signals within a second frequency range and to compress said audio signals within said second frequency range when the volume of said audio signals within said second frequency range is above a second threshold volume.

According to a second aspect of the present invention, there is provided a hearing protection method including steps (a) splitting incoming audio signals into at least audio signals within a first frequency range and audio signals within a second frequency range, (b) compressing said audio signals within said first frequency range when the volume of said audio signals within said first frequency range is above a first threshold volume, and (c) compressing said audio signals within said second frequency range when the volume of said audio signals within said second frequency range is above a second threshold volume.

A hearing protection device and a hearing protection method according to an embodiment of the present invention will now be described, by way of examples only, with reference to the accompany drawings, in which:
Fig. 1 is a perspective view of a hearing protection device according to an embodiment of the present invention;
Fig. 2 is a partly exploded perspective view of the hearing protection device shown in Fig. 1;
Fig. 3 is another perspective view of the hearing protection device shown in Fig. 1;
Fig. 4 is a perspective view of the hearing protection device shown in Fig. 1 with a cover removed;
Fig. 5A shows an ear tip attachable to the hearing protection device shown in Fig. 1, prior to molding;
Fig. 5B shows the ear tip shown in Fig. 5A after molding;
Fig. 6 shows the ear tip of Fig. 5A attached to the hearing protection device shown in Fig. 1, prior to molding;
Fig. 7 shows the ear tip of Fig. 5A attached to the hearing protection device shown in Fig. 1, after molding;
Fig. 8 shows a schematic diagram of a circuit of a prior art hearing protection device;
Fig. 9 shows a schematic diagram of a circuit of a hearing protection device according to an embodiment of the present invention;
Fig. 10 shows a graph showing an exemplary scheme of compressing incoming audio signals according to a first mode of operation of a hearing protection device according to an embodiment of the present invention;
Fig. 11 shows a graph showing an exemplary scheme of compressing incoming audio signals according to a second mode of operation of a hearing protection device according to an embodiment of the present invention; and
Fig. 12 is a circuit diagram of an exemplary charging circuit of a hearing protection device according to the present invention.

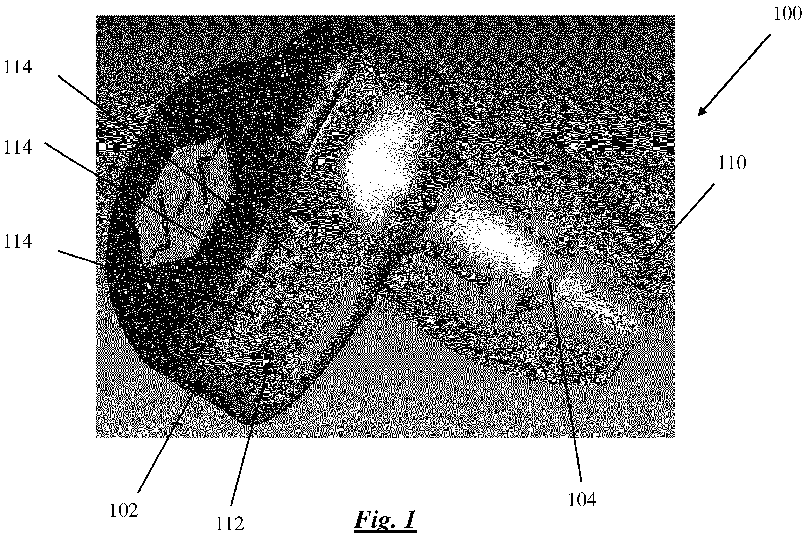

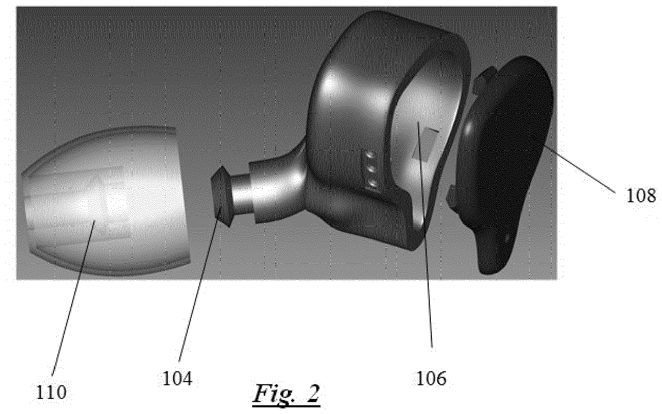

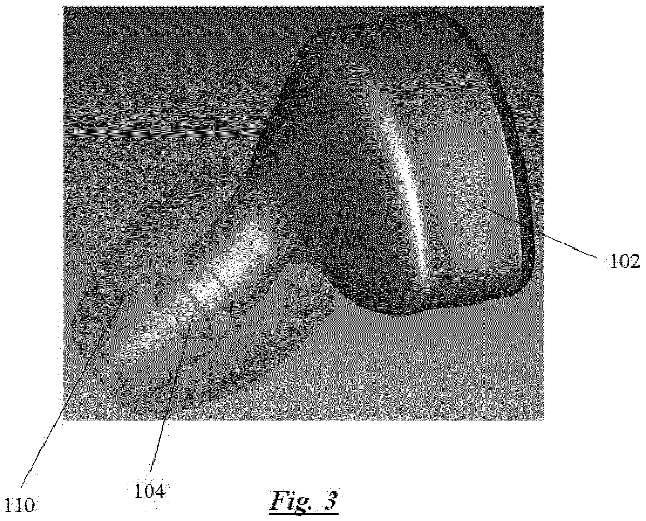

A hearing protection device according to an embodiment of the present invention is shown in Figs. 1 to 4, and generally designated as 100. The device 100 has a housing 102 an a speaker 104 connected with each other. The housing 102 has an internal space 106 which is covered by a cover 108 releasably engaged with the housing 102. The space 106 contains various electronic and electrical components of the device 100, e.g. one ore more rechargeable batteries, a micro-controller and/or an integrated circuit, in electrical connection with the speaker 104. The speaker 104 is surrounded by an ear tip 110, forming a part of an ear bud. Although the ear tip 110 is shown as being transparent in Figs. 1 and 3 to better show the shape of the speaker 104, the ear tip 110 may be opaque or translucent.

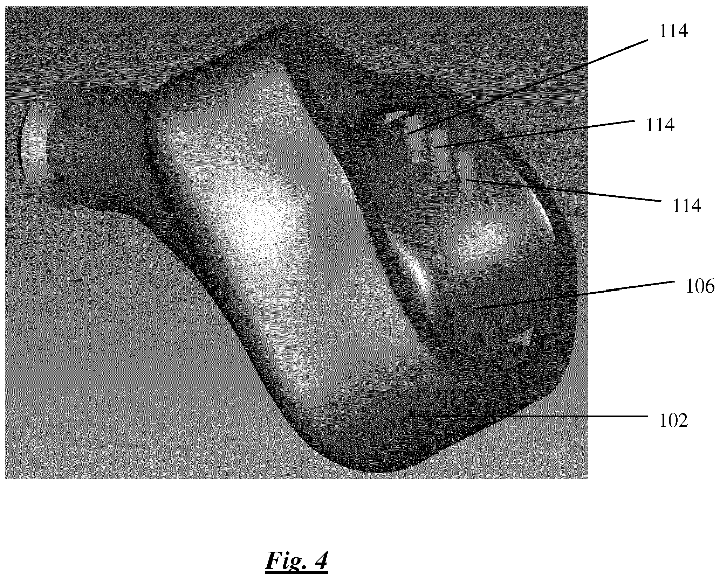

As shown more clearly in Figs. 1 and 4, three sockets 114 are provided through a wall 112 of the housing 102 of the device 100, which extend into the space 106 of the housing 102 of the device 100. The sockets 114 act as a multi-purpose connector, allowing, respectively, (i) connection with a data processing apparatus *via* a cable, allowing the programming of the micro-controller and/or an integrated circuit in the space 106 for adjusting the parameters and modes of operation of the device 100, (ii) connection with a mobile device (e.g. a smart phone) *via* a connection wire for receiving audio signals (e.g. music) therefrom, and (iii) connection with a charging cable for charging of one or more rechargeable batteries in the space 106.

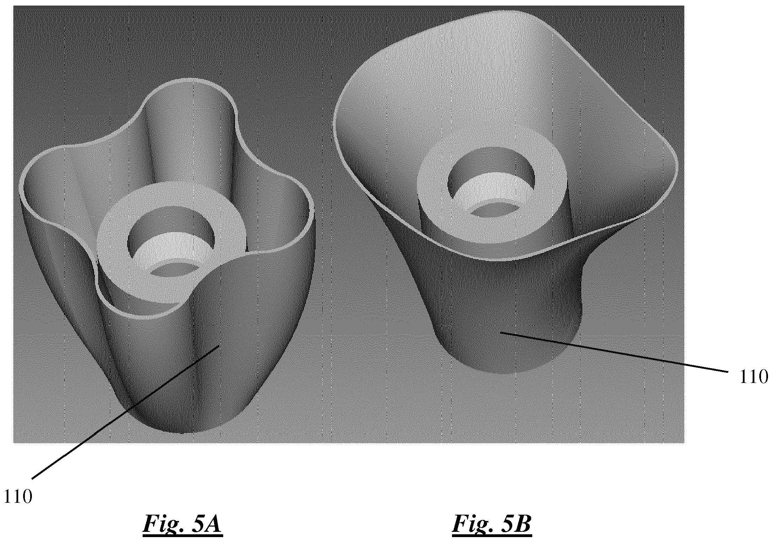

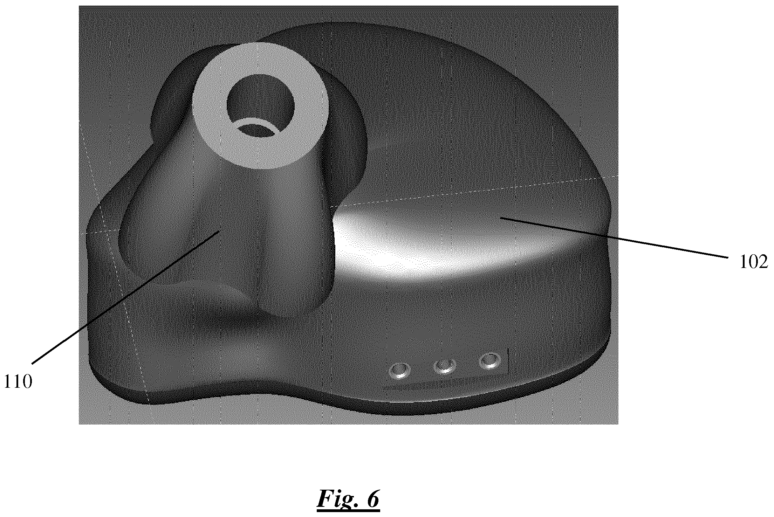

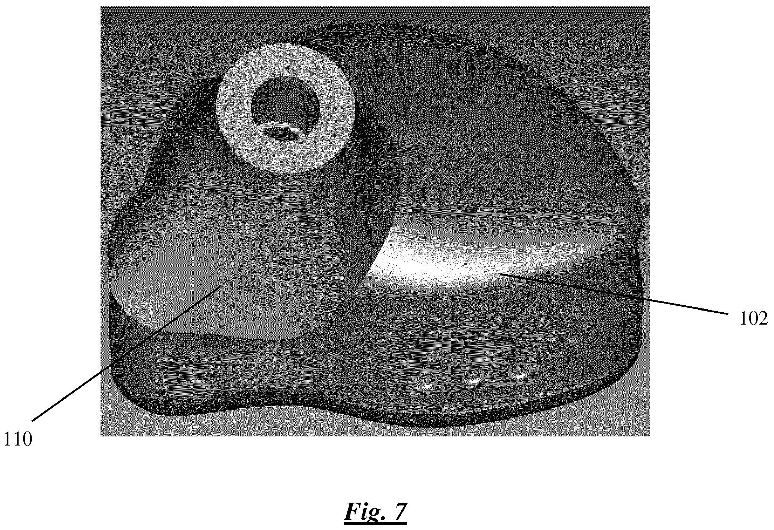

To adapt the device 100 to each specific user, custom ear-mold is designed for home fitting by the users without professional assistance. The ear tip 110 includes (a) a pleated ear tip and (b) two parts of "fast" cure silicone. The two parts of silicone are to be mixed well before use and is then molded in the ears of the user within 3 minutes. The centre tube (stem) of the ear tip 110 is plugged to prevent silicone from blocking sound. Pre-mixed silicone is then injected at the outer wall of the pleated tip 110. While the filled ear tips 110 are being molded and cured inside the ears, the shape of the ear canals are formed in about 5 minutes. Fig. 5A shows the ear tip 110 prior to molding, and Fig. 5B shows the ear tip 110 after molding. Fig. 6 shows the ear tip 110 prior to molding, as attached to the device 100, and Fig. 7 shows the ear tip 110 after molding, as attached to the device 100.

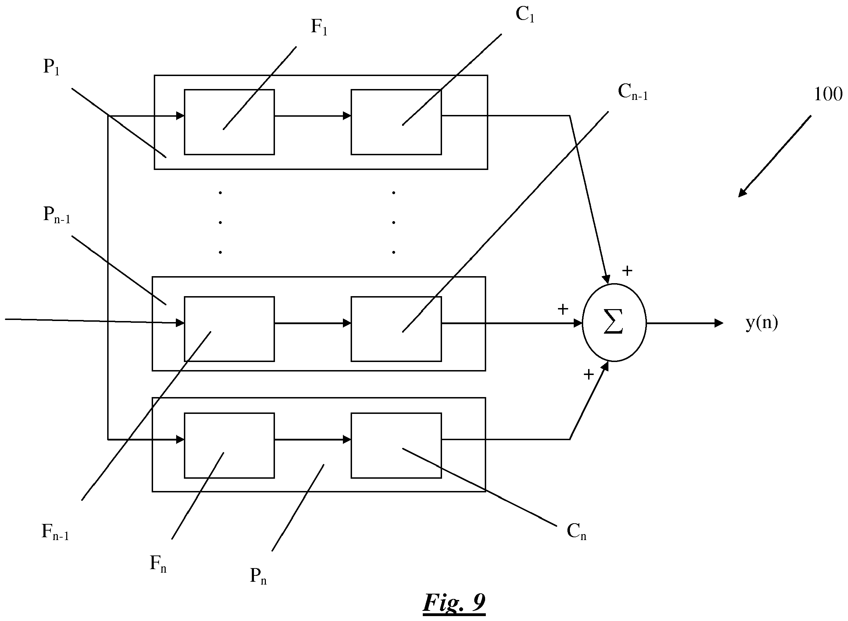

Fig. 9 shows a schematic diagram of a circuit of the hearing protection device 100. The device 100 includes a number of audio signal processors P₁, ... Pₙ₋₁, Pₙ arranged in parallel with one another. Each such audio signal processor P₁, ... Pₙ₋₁, Pₙ thus acts as a channel. Each audio signal processor (thus, each channel) includes a respective audio signal frequency filter F₁, ... Fₙ₋₁, Fₙ which passes audio signals within a specific frequency range and a respective audio signal compressor C₁, ... Cₙ₋₁, Cₙ arranged in series with the respective audio signal frequency filter F₁, ... Fₙ₋₁, Fₙ. The audio signal compressors C₁, ... Cₙ₋₁, Cₙ compress the audio signals within the respective frequency range when the volume of the audio signals within the specific frequency range is above a certain pre-determined threshold volume. The pre-determined threshold volume may be adjusted to be a sound pressure input of between 40 dB to 70 dB, and the channels may be pre-set with a same predetermined threshold volumes or different predetermined threshold volumes. By way of such an arrangement, the device 100 splits incoming audio signals into a number frequency ranges and each audio signal processor P₁, ... Pₙ₋₁, Pₙ is adapted to process audio signals within a specific frequency range.

In an embodiment, incoming audio signals are split into the following eight frequency ranges (bands):
Range 1 : < 250 Hz
Range 2: 250 Hz - 750 Hz
Range 3: 750Hz - 1,250 Hz
Range 4: 1,250 Hz - 1,750 Hz
Range 5: 1,750 Hz - 2,750 Hz
Range 6: 2,750 Hz - 3,750 Hz
Range 7: 3,750 Hz - 5,500 Hz
Range 8: > 5,500 Hz
For this purpose, the device 100 has eight audio signal processors P₁, ... P₇, P₈ arranged in parallel with one another. Each audio signal processor P₁, ... P₇, P₈ includes a respective audio signal frequency filter F₁, ... F₇, F₈ and a respective audio signal compressor C₁, ... C₇, C₈ arranged in series. As such, the audio signal frequency filter F₁ is a low-pass filter, the audio signal frequency filters F₂ to F₇ are band-pass filters, and the audio signal frequency filter F₈ is a high-pass filter.

The audio signals thus processed by the audio signal frequency filters F₁, ... F₇, F₈ and audio signal compressors C₁, ... C₇, C₈ of the device 100 are re-combined for subsequent output by the speaker 104 of the device 100.

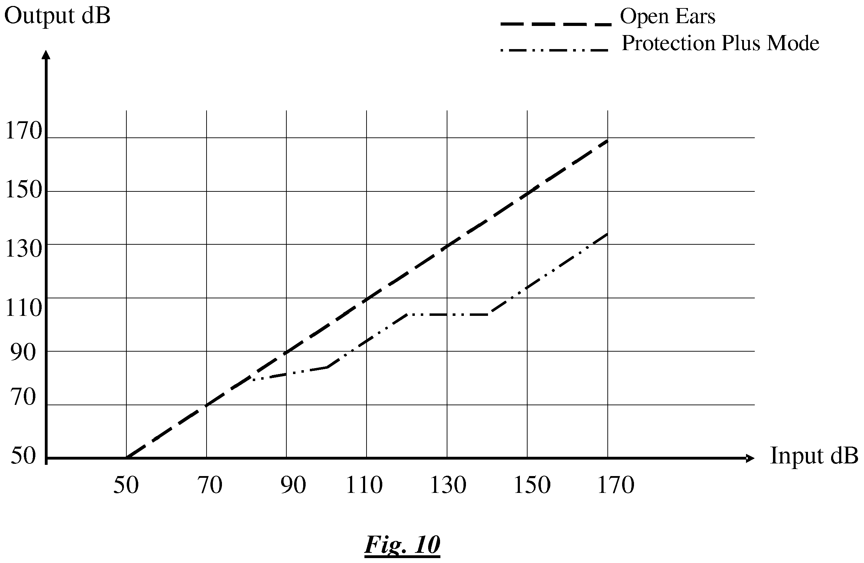

Fig. 10 is a graph showing an exemplary scheme of compressing incoming audio signals according to a first mode of operation of the hearing protection device 100, called "Protection Plus Mode". In this mode of operation, when the volume of audio signals passing through one of the signal frequency filters F₁, ... F₇, F₈, say the audio signal frequency filter F₁, exceeds 80 dB, the corresponding audio signal compressors C₁, ... C₇, C₈, say the audio signal compressor C₁, compresses the audio signals passed by the audio signal frequency filter F₁ according to the scheme shown in Fig. 10. In particular, it can be seen that, in this Protection Plus Mode, and taking the audio signals within the frequency range processed by the audio signal processor P₁ (with the filter F₁ and the audio signal compressor C₁) as an example:
(a) compression is carried out by the audio signal compressor C₁ when the volume of audio signals passing through the signal frequency filter F₁ is above 80 dB,
(b) when the volume is between 90 dB to 120 dB, the volume of the audio signals passing through the signal frequency filter F₁ is compressed by 15 dB from the open ear volume,
(c) when the volume is between 120 dB to 140 dB, the volume of the audio signals passing through the signal frequency filter F₁ is compressed to around 105 dB, and
(d) when the volume is above 140 dB, the volume of the audio signals is compressed by 35 dB from the open ear volume.
The "Protection Plus Mode" seeks to offer a high degree of hearing protection, typically suitable for use in very noisy environment.

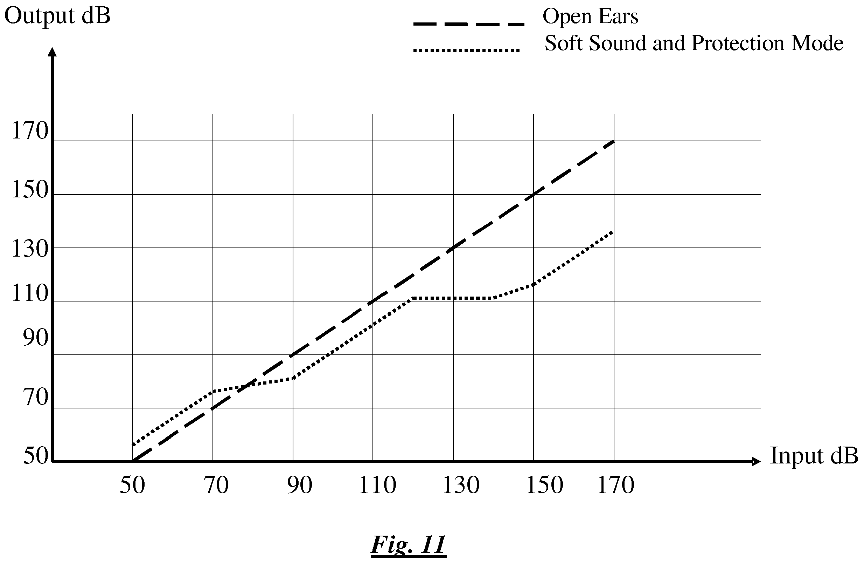

Fig. 11 is a graph showing an exemplary scheme of compressing incoming audio signals according to a second mode of operation of a hearing protection device 100, called "Soft Sound and Protection Mode". In this mode of operation, when the volume of audio signals passing through one of the signal frequency filters F₁, ... F₇, F₈, say the audio signal frequency filter F₁, exceeds 80 dB, the corresponding audio signal compressors C₁, ... C₇, C₈, say the audio signal compressor C₁, compresses the audio signals passed by the audio signal frequency filter F₁ according to the scheme shown in Fig. 11; and when the volume of audio signals passing through one of the signal frequency filters F₁, ... F₇, F₈, say the audio signal frequency filter F₁, is below 80 dB, the corresponding audio signal compressors C₁, ... C₇, C₈, say the audio signal compressor C₁, add gains to the audio signals. In particular, it can be seen that, in this Soft Sound and Protection Mode, and taking the audio signals within the frequency range processed by the audio signal processor P₁ (with the filter F₁ and the audio signal compressor C₁) as an example:
(a) a gain of 6 dB is added by the compressor C₁ when the volume of audio signals passing through the signal frequency filter F₁ is below 70 dB,
(b) when the volume of audio signals passing through the signal frequency filter F₁ is between 70 dB and 90 dB, the output volume is maintained at roughly 80 dB,
(c) when the volume of audio signals passing through the signal frequency filter F₁ is between 90 dB and 120 dB, the volume is compressed by 9 dB from the open ear volume,
(d) when the volume of audio signals passing through the signal frequency filter F₁ is between 120 dB and 140 dB, the volume is compressed at 110 dB, and
(e) when the volume of audio signals passing through the signal frequency filter F₁ is above 140 dB, the volume is compressed by 35 dB from the open ear volume.
The "Soft Sound and Protection Mode" enables users to pay attention to the ambient, like dropping pins, and singing birds. This feature is useful for such users as hunters, who need ear protection against gun shooting noise, while loss of sound awareness could be dangerous. Ear protection will gradually phase in when the volume of the input noise increases. When the volume of the input sound reaches a relatively high level, e.g. 120 dB, the device 100 will instantly compress the volume to a safe level.

The above only illustrates examples of possible compression ratios by which audio signals of each split frequency range may be compressed. The compression ratio of each split frequency range may be adjusted, as follows: 1:1; 1.05:1; 1.11:1; 1.18:1; 1.25:1; 1.33:1; 1.43:1; 1.54:1; 1.67:1; 1.82:1; 2:1; 2.22:1; 2.5:1; 2.86:1; 3.33:1; 4:1.

The transient response of each channel would react according to the average power in each channel calculated every hundreds of micro-seconds. The device 100 automatically switches between Normal Mode and Fast Mode in order to react with the proper compression requirements.

In Normal Mode, the overall volume is reduced by compressing the peaks of the sound signals. Compression takes place when the volume of the sound is over a certain threshold volume, at which point only the overshoot will be compressed. User's ears are protected by pushing down the peaks of a signal and reducing the volume fluctuations. Typical use of this Normal Mode includes busy streets, restaurants and playground. While the overall sound level is reduced, the original sound quality and integrity are maintained.

In Fast Mode, when a sound overshoots the volume threshold, the compressors do not instantly apply the full compression to the overshooting signal. Instead, the compression ratio increases from 1:1 to the maximum within a time period called the "attack time". Similarly, when the volume of the sound drops back below the threshold, the compressors do not instantly stop compressing the sound signals. Instead, the compression ratio is gradually reduced from the compression ratio until it reaches 1:1, again within a time period called the "release time". Applying compression with extremely short attack time will cause the signals to be almost instantly compressed with the maximum ratio when the level overshoots the threshold volume. Fast Mode will protect ears from extremely loud and sudden sound, such as drum, bass guitar, gun shooting, hammer, rock concert. The device 100 may be programmed for specific applications for different markets and users. For a bass guitar, the compressed "Fast Mode" will apply a short attack time (e.g. ∼ 10-20 ms) but a long release time before the next node is struck, thus creating a good sustaining mood.

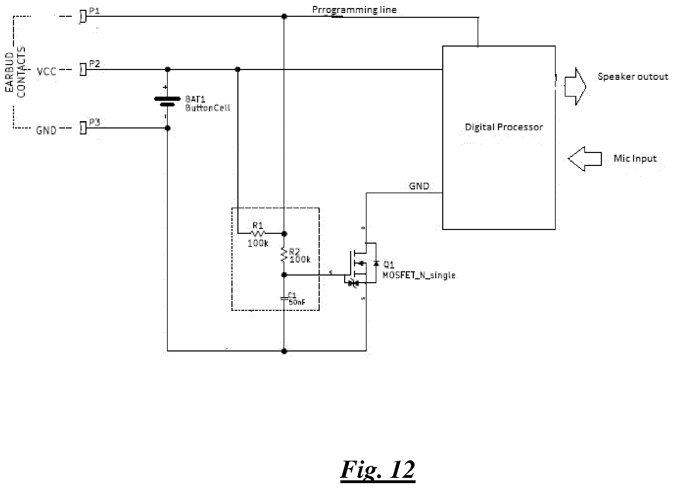

Turning to Fig. 12, which shows a circuit diagram of an exemplary charging circuit of a hearing protection device according to the present invention. In the hearing protection device 100, there is no power switch (or so called "ON/OFF switch"), so as to simplify usr operation, and to reduce the space required for the electrical and/or electronic components. More importantly, there is no commercially available ON/OFF switch suitable for use in the hearing protection device 100.

In the hearing protection device 100 with the three sockets 114, the hearing protection device 100 is OFF during recharging, and the device 100 is ON during normal use and programming. During normal use, a metal-oxide-semiconductor field-effect transistor (MOSFET) gate Q1 of the circuit will pull high that provides a ground path so that the battery in the hearing protection device 100 will supply electrical current (e.g. electrical power) to the hearing protection device 100. During recharging, the MOSFET gate Q1 will pull low that removes the ground path to the hearing protection device 100. The hearing protection device 100 is thus power-off. During programming, it is important to keep the hearing protection device 100 ON. The MOSFET gate capacitor C1 is large enough to filter programming signal pulses and thus to maintain a positive gate voltage, thus ensuring that the hearing protection device 100 is ON.

It should be understood that the above only illustrates examples whereby the present invention may be carried out, and that various modifications and/or alterations may be made thereto without departing from the spirit of the invention.

It should also be understood that certain features of the invention, which are, for clarity, described in the context of separate embodiments, may be provided in combination in a single embodiment. Conversely, various features of the invention which are, for brevity, described in the context of a single embodiment, may also be provided separately or in any appropriate sub-combinations.

## Claims

1. A hearing protection device including:
at least a first audio signal processor and a second audio signal processor arranged in parallel with one another,
wherein said first audio signal processor is adapted to pass audio signals within a first frequency range and to compress said audio signals within said first frequency range when the volume of said audio signals within said first frequency range is above a first threshold volume, and
wherein said second audio signal processor is adapted to pass audio signals within a second frequency range and to compress said audio signals within said second frequency range when the volume of said audio signals within said second frequency range is above a second threshold volume.

2. A device according to Claim 1, wherein said first threshold volume and said second threshold volume are different.

3. A device according to Claim 1 or 2,
wherein said first audio signal processor is adapted to compress said audio signals within said first frequency range by a first ratio when the volume of said audio signals within said first frequency range is above said first threshold volume, and
wherein said second audio signal processor is adapted to compress said audio signals within said second frequency range by a second ratio which is different from said first ratio when the volume of said audio signals within said second frequency range is above said second threshold volume.

4. A device according to any one of Claims 1 to 3, wherein said first audio signal processor is adapted to amplify said audio signals within said first frequency range by a third ratio when the volume level of said audio signals within said first frequency range is below a third threshold volume which is different from said first threshold volume, or optionally wherein said first audio signal processor is adapted to amplify said audio signals within said first frequency range by a third ratio when the volume level of said audio signals within said first frequency range is below said first threshold volume.

5. A device according to any of the preceding claims, including eight said audio signals processors connected arranged in parallel with one another.

6. A device according to any of the preceding claims, further including means for recombining audio signals processed by said at least first audio signal processor and said second audio signal processor.

7. A device according to any of the preceding claims, wherein a time duration of an attack phase of at least said first audio signal processor is adjustable, or optionally wherein a time duration of a release phase of at least said first audio signal processor is adjustable.

8. A device according to any of the preceding claims, further including a first socket for detachable connection with a mobile device, or optionally further including a second socket in connection with an internal battery, or optionally further including a third socket in connection with a central processing unit.

9. A device according to any of the preceding claims, further including a charging circuit adapted to ensure that said device is ON during charging, and optionally wherein said charging circuit includes a metal-oxide-semiconductor field-effect transistor (MOSFET) gate connected with a capacitor.

10. A hearing protection method including steps:
(a) splitting incoming audio signals into at least audio signals within a first frequency range and audio signals within a second frequency range,
(b) compressing said audio signals within said first frequency range when the volume of said audio signals within said first frequency range is above a first threshold volume, and
(c) compressing said audio signals within said second frequency range when the volume of said audio signals within said second frequency range is above a second threshold volume.

11. A method according to Claim 10,
wherein said audio signals within said first frequency range are compressed in said step (b) by a first ratio when the volume of said audio signals within said first frequency range is above said first threshold volume,
wherein said audio signals within said second frequency range are compressed in said step (c) by a second ratio which is different from said first ratio when the volume of said audio signals within said second frequency range is above said second threshold volume, and
wherein said first ratio is different from said second ratio.

12. A method according to Claim 10 or 11, further including a step (d) of amplifying said audio signals within said first frequency range by a third ratio when the volume of said audio signals within said first frequency range is below a third threshold volume which is different from said first threshold volume.

13. A method according to Claim 10 or 11, further including a step (e) of amplifying said audio signals within said first frequency range by a third ratio when the volume of said audio signals within said first frequency range is below said first threshold volume.

14. A method according to any one of Claims 10 to 13, wherein, in said step (a), incoming audio signals are split into audio signals within eight frequency ranges.

15. A method according to any one of Claims 10 to 14, further including, after said step (d), a step (f) of recombining said audio signals, or optionally including a step (g) of adjusting an attack time of at least said step (b), or optionally including a step (h) of adjusting a release phase of at least said step (b).
